# EUROPÄISCHE PATENTANMELDUNG

(11) **EP 2 308 503 A1**
(43) Veröffentlichungstag der Anmeldung: **13.04.2011**
(21) Anmeldenummer: 10460010.1
(22) Anmeldetag: 09.03.2010
(51) Int. Cl.: A61K 38/22

(54) **Verfahren zur Beschleunigung der Entwicklung von Nutzsäugetieren**

(30) Priorität: 24.09.2009 PL 38911609
(71) Anmelder: Biochefa, 41-205 Sosnowiec (PL)
(72) Erfinder: Ryszka, Florian, 41-207 Sosnowiec (PL); Dolinska, Barbara, 40-282 Katowice (PL); Leszczynska, Lucyna, 41-200 Sosnowiec (PL); Smorag, Zdzislaw, 32-083 Balice (PL); Gajda, Barbara, 31-115 Krakow (PL); Szczesniak-Fabianczyk, Barbara, 30-108 Krakow (PL); Koska, Miroslaw, 46-250 Wolczyn (PL); Kowalski, Waldemar, 50-382 Wroclaw (PL)
(74) Vertreter: Bocionek, Karol

(57) **Zusammenfassung**

Verfahren der Beschleunigung der Entwicklung von Nutzsaugtieren, das in einem Verabreichen der enthaltenden Prolaktin Präparate besteht, in dieser Weise, dass man den neugeborenen Nutzsaugtieren durch den Mund oder subkutan oder intravenös oder rektal, jedoch vorteilhafter durch den Mund während der Säugzeit, Prolaktin vorzugsweise eigener Gattung in der Menge von 0,01 mg bis 1 mg, vorteilhaft von 0,05 mg bis 0,06 mg pro Kilogramm der Körpermasse enthaltendes Präparat in wässrig - isotonischer Lösung zu verabreichen ist.

## Beschreibung

Die Erfindung betrifft eines Verfahren zur Beschleunigung der Entwicklung in Züchtung von Haushaltstieren.

Bei der Entwicklung von Säugern ist sehr wichtig den Tieren die Bedingungen einer regelmäßigen Entwicklung und Zunehmung des Körpermasse und Zahl der aufgezogenen Nachwuchs zu schaffen.

Einen von wichtigen, bedingenden Entwicklung und Wachstum von neugeborenen Säugern Faktoren ist Hormon der Hirnanhangdrüse - Prolaktin.

Prolaktin verwendet man in Falle der Laktationstörung, die in einen Schwund (Agalaktie) oder eine Verminderung der Erzeugung oder Milchabsonderung (Hippolaktation) durch Weibchenmilchdrüse in Ernährungszeit von Nachwuchs besteht.

Es gibt Art und Weise bekannt, die Entwicklung und Wachstum der Zahl von aufgezogenen Tieren zu beschleunigen, die in Stimulierung der Laktation von Füttermütter durch Verabreichen der enthaltenden Prolaktin - Präparate bestehen.

Es ist eine Laktationstimulierung bei Kühe, Ziegen, Schafe, Saue und Stuten bekannt, die auf einem intramuskulär Verabreichen am Tag der Geburt oder nach der Geburt ein Prolaktin enthaltenden Präparat, oder der entsprechenden Hormonen, als auch der anderen, stimulierenden Laktation Präparate beruht.

Es ist vom polnischen Patentanmeldung P-386294 ein Verfahren der Beschleunigung der Entwicklung von Ferkeln bekannt, das in Verabreichen einmalig durch den Mund Prolaktin enthaltendes Präparat in wässrig - isotonischer Lösung, besteht.

Der Erfindung gemäß, das Wesen besteht darin, dass neugeborenen Säugern von Nutztieren durch den Mund oder subkutan oder rektal oder intravenös während der Säugezeit ein Prolaktin vorzugsweise eigener Gattung in einer Menge von 0,01 mg bis 1 mg, vorteilhaft von 0,05 mg bis 0,6 mg, pro 1 kg Körpermasse enthaltendes Präparat in wässrig - isotonischer Lösung zu verabreichen ist.

Das Verfahren nach der Erfindung verursacht die Beschleunigung der Entwicklung von jungen Tieren, vergrößernd ihre Körpermasse bis 40 % und vermindernd die Zahl von Tierverrecken und als Endresultat ist Ertrag von größeren Tierzuwachs.

### Beispiel 1.

Den Saugferkeln am 20 Tag nach der Geburt mit einem Körpermasse von 4 kg wird per Mund 8 mg eines Präparates mit 0,1 mg Prolaktin in 1 ml der Lösung durch Auflösen von 100 mg Prolaktin in 500 ml 0,9 % iger Natriumchlorid-Lösung, mit Konservierungsmittel und ergänzend um 0,9 % Natriumchlorid -Lösung auf 1000 ml, verabreicht. Durchschnittliche Ferkel - Massenzunahme war auf einem Niveau von 185 Gramm, während in einer Kontrollgruppe, erhaltende als Placebo 0,9 % Natriumchlorid- Lösung, die Massenzunahme betrug 160 g pro Tag.

### Beispiel 2

Den Saugkalben am 3 Tag nach der Geburt mit einem Körpermasse von 4,5 kg wird subkutan 2 ml. eines Präparates mit 0,5 mg Prolaktin in 1 ml der Lösung, durch Auflösen 500 mg Prolaktin in 500 ml 0,9% iger Natriumchlorid - Lösung, mit Konservierungsmittel und ergänzend um 0,9 % Natriumchlorid - Lösung auf 1000 ml, verabreicht. Nach 30 Tagen seit dem Verabreichen des Präparates erhielt man 115 Gramm täglichen Körpermassenzunahme und in Kontrollgruppe als Placebo erhaltenden 0,9 % Natriumchlorid - Lösung betrug die Massenzunahme von 85 Gramm. Es wurde kein Verrecken von Tieren bestätigt.

### Beispiel 3.

Den Saugkalben am 30 Tag nach der Geburt wird rektal in Form eines Stuhlzäpfchens ein Präparat mit 2,0 mg Prolaktin, durch Auflösen 200 mg Prolaktin in 20 ml 0,9 % iger Natriumchlorid -Lösung, hinzugebend 180 Gramm Witepsol und zu Zäpfchen verformt mit einer Masse von 1 g, verabreicht. Nach 30 Tagen seit dem Verabreichen des Präparates erhielt man den täglichen Zuwachs der Körpermasse um 15 % höher als in Vergleich zu der erhaltenden als Placebo 0,9 % Natriumchlorid-Lösung Kontrollgruppe.

### Beispiel 4.

Den neugeborenen Zickleinen wird intramuskulär 0,5 ml des Präparates mit 0,6 mg Prolaktin in 1 ml der Lösung durch Auflösen 200 mg Prolaktin in 500 ml 0,9 % iger Natriumchlorid- Lösung, mit Konservierungsmittel, ergänzend um 0,9 % Natriumchlorid - Lösung auf 1000 ml verabreicht. Nach 25 Tagen seit dem Verabreichen erhielt man die täglichen Massenzunahme von 170 g in Vergleich zu der als Placebo erhaltenden 0,9 % Natriumchlorid-Lösung Kontrollgruppe, bei denen die tägliche Massenzunahme von 141 g betrug.

### Beispiel 5

Einer Gruppe von neugeborenen Kälbern mit einer Körpermasse von 40 kg wird per Mund 40 ml des Präparates mit 0,2 mg Prolaktin in 1 ml der Lösung durch Auflösen 200 mg Prolaktin in 500 ml 0,9 % iger Natriumchlorid-Lösung mit Konservierungsmittel und ergänzend um 0,9 % Natriumchlorid -Lösung auf 1000 ml, verabreicht. Nach 21 Tagen seit dem Verabreichen des Präparates erhielt man durchschnittlich die täglichen Zuwachs der Körpermasse um 22 % höher, als in der als Placebo erhaltenden 0,9 % Natriumchlorid- Lösung Kontrollgruppe. Die Tiere zeigten auch besserer biochemischer Eigenschaften von Blut aus, und waren widerstandfähiger gegen negative Faktoren.

### Beispiel 6.

Einer Gruppe von Fohlen am 2 Tag nach der Geburt wird intravenös 5,0 ml des Präparates mit 2,0 mg Prolaktin in 1 ml der Lösung, durch Auflösen 200 mg Prolaktin in 1000 ml 0,9 % iger Natriumchlorid-Lösung verabreicht. Hingegen der Kontrollgruppe wird man derselbe Menge der 0,9 % igen physiologischen Salz- Lösung verabreicht. Nach 50 Tagen seit dem Verabreichen des Präparates wurde man tägliche Körpermassenzunahme um 27 % höher als in Vergleich zu Kontrollgruppe bestätigt. Die Fohlen haben bessere Widerstandfähigkeit gegen Krankheiten, bessere Eigenschaften des Blutes und bessere Verdaulichkeit des Futters ausgezeigt.

## Patentansprüche

1. Verfahren zur Beschleunigung der Entwicklung von Nutzsaugtieren, das in einem Verabreichen der enthaltenden Prolaktin Präparate besteht, **dadurch gegenzeichnet, dass** neugeborenen Nutzsaugtieren durch den Mund oder subkutan oder intravenös oder rektal, jedoch vorteilhafter durch den Mund während der Säugzeit, ein Prolaktin vorzugsweise eigener Gattung in einer Menge von 0,01 mg bis 1 mg, vorteilhaft von 0,05 mg bis 0,06 mg pro Kilogramm des Körpermasse enthaltendes Präparat in wässerig - isotonischer Lösung zu verabreichen ist.
